# EUROPÄISCHE PATENTANMELDUNG

(11) **EP 0 965 590 A1**
(43) Veröffentlichungstag der Anmeldung: **22.12.1999**
(21) Anmeldenummer: 99111671.6
(22) Anmeldetag: 16.06.1999
(51) Int. Cl.: C07D 233/64, C07D 233/68

(54) **Verfahren zur Herstellung von Formylimidazolen**

(30) Priorität: 18.06.1998 EP 98111174
(71) Anmelder: LONZA A.G., CH-4002 Basel (CH)
(72) Erfinder: Mettler, Hanspeter, Dr., 3930 Visp (CH); Hanselmann, Paul, Dr., 3902 Brig-Glis (CH)
(74) Vertreter: Winter, Brandl, Fürniss, Hübner, Röss, Kaiser, Polte, Kindermann Partnerschaft

(57) **Zusammenfassung**

Beschrieben wird ein neues Verfahren zur Herstellung von Formylimidazolen der allgemeinen Formel 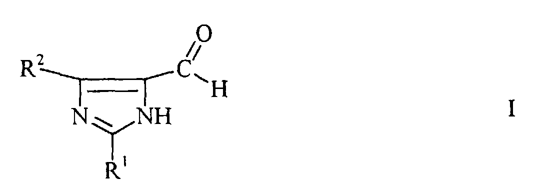 oder deren Tautomeren, worin R¹ Wasserstoff oder Alkyl und R² Wasserstoff, Halogen oder Alkyl bedeutet, bei dem in einer ersten Stufe ein Imidazolderivat der allgemeinen Formel 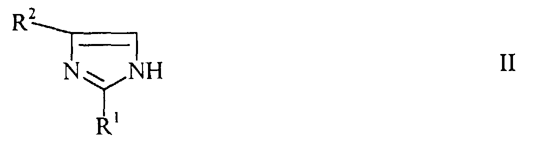 oder dessen Tautomere, worin R¹ und R² die genannte Bedeutung haben, durch Einführen einer Aminoschutzgruppe in ein Imidazolderivat der allgemeinen Formel 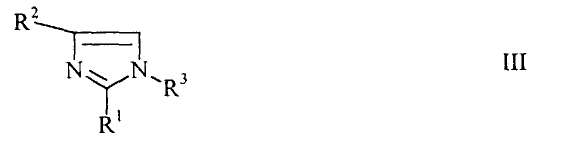 oder dessen Tautomere, worin R³ eine Aminoschutzgruppe bedeutet, überführt werden, diese in einer zweiten Stufe in Gegenwart einer Organometall-Verbindung und eines geeigneten Elektrophils in ein Imidazolderivat oder allgemeinen Formel 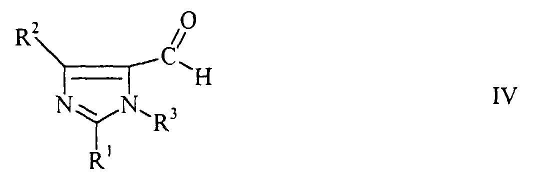 oder dessen Tautomere, worin R¹, R² und R³ die genannte Bedeutung haben, formyliert werden und dann in einer dritten Stufe durch Abspalten der Aminoschutzgruppe das Endprodukt der Formel I erhalten wird.

## Beschreibung

Die vorliegende Erfindung betrifft ein neues Verfahren zur Herstellung von Formylimidazolen der allgemeinen Formel 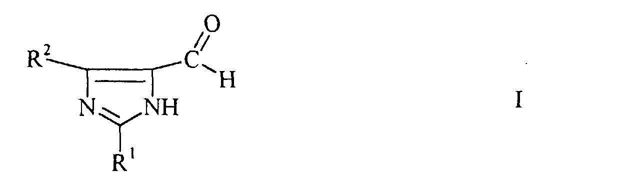 oder deren Tautomeren, worin R¹ Wasserstoff oder Alkyl und R² Wasserstoff, Halogen oder Alkyl bedeutet.

Formylimidazole sind wichtige Zwischenprodukte zur Herstellung von pharmazeutischen Wirkstoffen wie z. B. für Diuretika oder Antihypertonika (WO 92/20651).

Ein Verfahren zur Herstellung von Formylimidazolen wird bspw. in der CH-A-685 496 beschrieben. Hierbei werden Hydroxymethylimidazole durch katalytische Oxidation in Gegenwart von Edelmetallkatalysatoren wie Platinwismuth, Platinschwarz, Platin oder Palladium auf Aktivkohle unter Einblasen von Sauerstoff zu den Formylimidazolen oxidiert. Nachteilig bei diesem Verfahren sind lange Reaktionszeiten und die Bildung von Nebenprodukten.

Aufgabe der vorliegenden Erfindung war daher ein ökonomischeres Verfahren zur Herstellung von Formylimidazolen zur Verfügung zu stellen, mit welchem die Produkte in hoher Reinheit isoliert werden können.

Diese Aufgabe wird durch das Verfahren nach Anspruch 1 gelöst.

Alkyl wird im folgenden als eine geradkettige oder verzweigte Alkylgruppe definiert. Zweckmässig bedeutet Alkyl C₁₋₆-Alkyl wie Methyl, Ethyl, Propyl, Isopropyl, Butyl, Isobutyl, tert-Buytl, Pentyl und seine Isomere sowie Hexyl und seine Isomere. Vorzugsweise bedeutet Alkyl Butyl.

### Halogen wird im folgenden als F, Cl, Br, J definiert. Vorzugsweise bedeutet Halogen Cl

Der Begriff "Tautomere" bedeutet, dass die Verbindungen sich lediglich in der Position einer beweglichen Gruppe und in der Lage einer Doppelbindung unterscheiden. Tautomere Verbindungen der allgemeinen Formel I können demnach sein 4- oder 5-Formylimidazole wie 2-Alkyl-4-formyl-5-alkyl-imidazole, 4-Formyl-5-alkylimidazole, 4-Formyl-5-halogen-imidazole, 2-Alkyl-4-formyl-5-halogen-imidazole, 2-Alkyl-4-alkyl-5-formyl-imidazole, 2-Alkyl-4-halogen-5-formyl-imidazole, 4-Alkyl-5-formyl-imidazol und 4-Halogen-5-formyl-imidazole. Tautomere Verbindungen der allgemeinen Formel II und III sind zweckmässig die entsprechenden nichtformylierten Verbindungen und tautomere Verbindungen der allgemeinen Forme IV sind die entsprechenden formylierten N-geschützten Verbindungen. Die bevorzugten Verbindungen der allgemeinen Formel I sind 2-Butyl-4-formyl-imidazol und 2-Butyl-5-formylimidazol.

In der ersten Stufe des erfindungsgemässen Verfahrens wird ein Imidazolderivat der allgemeinen Formel 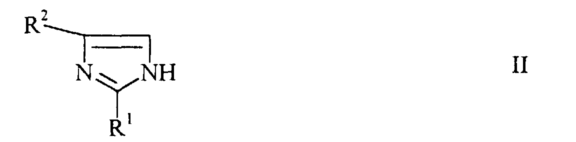 oder dessen Tautomere, worin R¹ und R² die genannte Bedeutung haben, durch Einführen einer Aminoschutzgruppe in ein Imidazolderivat der allgemeinen Formel 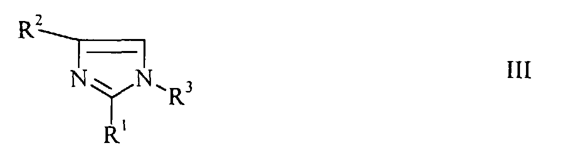 oder dessen Tautomere, worin R³ eine Aminoschutzgruppe bedeutet, überführt.

Die Imidazolderivate der allgemeinen Formel II (Edukte), wie z.B. das 2-Butylimidazol, sind käufliche Verbindungen oder können auf bekannte Weise beispielsweise gemäss US-A-2847417 oder gemäss J.L. Hughey, Synth. 1980, 489 hergestellt werden.

Als Aminoschutzgruppen können die fachmännisch üblichen dienen, wie Dialkylaminomethyl, Dialkylaminosulfonyl, Alkylsulfonyl, Alkoxymethyl, 1-Alkoxyethyl, Dialkoxymethyl, Aryloxycarbonyl, aliphatisches Oxycarbonyl, Piperidinomethyl und Toluolsulfonyl. Als Dialkylaminomethyl kann z.B. Dimethylaminomethyl, Dimethylaminoethyl, Dimethylaminopropyl dienen. Als Dialkylaminosulfonyl kann z.B. Dimethylaminosulfonyl oder Diethylaminosulfonyl dienen. Als Akylsulfonyl kann z.B. Methyl-, Ethyl-, Propyl-, oder Butylsulfonyl dienen. Als Alkoxymethyl kann beispielsweise Methoxymethyl, Ethoxymethyl oder Propoxymethyl und als Dialkoxymethyl kann Dimethoxymethyl, Diethoxymethyl oder Dipropoxymethyl dienen. Für 1-Alkoxyethyl seien beispielsweise 1-Methoxyethyl, 1-Ethoxyethyl und 1-Propoxyethyl erwähnt. Als Aryloxycarbonyl kann z.B. Phenyloxycarbonyl dienen. Als aliphatisches Oxycarbonyl kann beispielsweise Z (Benzyloxycarbonyl), BOC (Tert-Butoxycarbonyl) oder FMOC (Fluorenylmethoxycarbonyl) dienen. Vorzugsweise dienen als Aminoschutzgruppen Piperidinomethyl, Dialkylaminomethyl oder Dialkylaminosulfonyl.

Wie fachmännisch bekannt, werden diese Aminoschutzgruppen mittels den entsprechenden Schutzgruppenreagentien eingeführt Als Schutzgruppenreagentien können beispielsweise Dimethylamin/Formaldehyd, Piperidin/Formaldehyd, Bis(dimethylamino)methan, N,N-Dimethylsulfaminsäurechlorid, Ameisensäureorthoester, BOC-Cl, Z-Cl, Chlormethylethylether oder Dimethoxymethan eingesetzt werden.

Wie fachmännisch bekannt, ist die Wahl der Reaktionstemperatur, die Wahl des Lösungsmittels und die des pH-Wertes von der einzuführenden Aminoschutzgruppe abhängig und kann der entsprechenden Fachliteratur, wie z.B. Houben Weyl, Methoden der organischen Chemie, Band 15 (1-2), Thieme Verlag Stuttgart, 1974, entnommen werden.

Bei Anwendung der bevorzugten Aminoschutzgruppen Piperidinomethyl und Dialkylaminomethyl (Herstellung von Piperidinomethyl- und Dialkylaminomethylimidazol aus dem entsprechenden Amin und Formaldehyd) wird die Umsetzung in der ersten Stufe zweckmässig bei einer Temperatur von 0 bis 100°C, vorzugsweise bei einer Temperatur von 15 bis 50°C durchgeführt. Der pH-Wert liegt zweckmässig bei einem pH von 0 bis 8, vorzugsweise von 3 bis 6. Geeignete Lösungsmittel sind dann Wasser, polar protische oder aprotische Lösungsmittel wie Tetrahydrofuran, Methylenchlorid, Aceton, niedere Alkohole wie Methanol, Ethanol, Propanol oder Gemische von diesen mit Wasser. Bevorzugt ist Wasser das Lösungsmittel.
Wird beispielsweise Dialkylaminomethylimidazol aus dem entsprechenden Bis(dialkylamino)methan hergestellt, wird die Umsetzung zweckmässig bei einer Temperatur von 20 bis 100 °C, vorzugsweise bei einer Temperatur von 60 bis 90°C, durchgführt. Als Lösungsmittel können dann polar aprotische Lösungsmittel wie Tetrahydrofuran, Dioxan, Dimethoxymethan, Dimethylformamid, Dimethylsulfoxid, Acetonitril oder Methylenchlorid eingesetzt werden.

Bei Anwendung der bevorzugten Aminoschutzgruppe Dialkylaminosulfonyl (Herstellung von Dialkylaminosulfonylimidazol aus Dialkylsulfaminsäurechlorid oder Sulfuryldiimidazol und Sulfurylchlorid) wird die Umsetzung zweckmässig bei einer Temperatur von 0 bis 60°C, vorzugsweise bei einer Temperatur von 20 bis 40°C, durchgeführt. Zweckmässig wird diese Umsetzung in Gegenwart einer Base durchgeführt. Als Base kann ein Trialkylamin wie Triethylamin, ein Akali- oder Erdalkalimetallcarbonat wie Natrium- oder Kaliumcarbonat, ein Alkali- oder Erdalkalimetallhydroxid wie Natrium- oder Kaliumhydroxid oder das entsprechende Imidazol eingesetzt werden. Wird beispielsweise das entsprechende Imidazol als Base verwendet, wird dies im Überschuss eingesetzt. Bei Anwendung einer anderen der aufgeführten Basen werden zweckmässig 1 bis 2 Equivalente eingesetzt. Vorzugsweise wird als Base ein Trialkylamin in 1 bis 1,2 Equivalenten eingesetzt. Als Lösungsmittel können beispielsweise polar aprotische Lösungsmittel wie Tetrahydrofuran, Dioxan, Dimethoxymethan, Dimethylformamid, Dimethylsulfoxid, Acetonitril und Methylenchlorid verwendet werden.

In der zweiten Verfahrensstufe wird das Imidazolderivat der allgemeinen Formel III in Gegenwart einer Organometall-Verbindung und eines geeigneten Elektrophils in ein Imidazolderivat der allgemeinen Formel 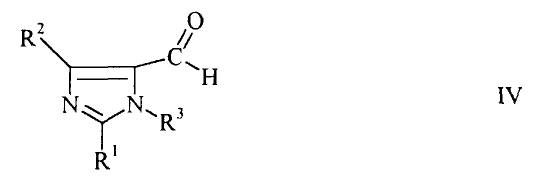 oder dessen Tautomere, worin R¹, R² und R³ die genannte Bedeutung haben, formyliert.

Mögliche Organometall-Verbindungen sind Alkylmetall-Verbindungen, worin Alkyl wie bereits beschrieben definiert ist, beispielsweise Alkylalkalimetall-Verbindungen, Alkylaluminium- oder Alkylmagnesium-Verbindungen oder Verbindungen wie Alkalimetallalkylamide. Als Alkylalkalimetall-Verbindungen können Alkyllithium, Alkylnatrium und Alkylkalium eingesetzt werden. Geeignete Alkyllithium-Verbindungen sind Methyl-,Ethyl-,Propyl- oder Butyllithium, geeignete Alkylnatrium-Verbindungen sind Methyl-, Ethyl-, Propyl- oder Buytlnatrium und geeignete Alkylkalium-Verbindungen sind Methyl-, Ethyl-, Propyl- oder Butylkalium. Als Alkalimetallalkylamid kann z.B. Lithiumdiisopropylamid eingesetzt werden.

Als Elektrophil werden Verbindungen mit einer Formylabgangsgruppe eingesetzt, beispielsweise N,N-Dialkylformamid oder Ameisensäurealkylester. Als N,N-Dialkylformamid können N,N-Dimethylformamid, N,N-Diethylformamid, N,N-Dipropylformamid oder N,N-Dibutylformamid verwendet werden. Als Ameisensäurealkylester sind Methyl-, Ethyl-, Propyl- oder Butylformiat geeignet. Insbesondere wird als Elektrophil Methylformiat verwendet.

Zweckmässig wird die Formylierung in der zweiten Stufe bei einer Temperatur von -100 bis 50°C, vorzugsweise bei einer Temperatur von -70 bis 20°C durchgeführt. Geeignete Lösungsmittel für die zweite Stufe sind aprotische Lösungsmittel wie Diethylether, Tetrahydrofuran, Methylenchlorid oder Hexan.

In der dritten Stufe wird das Imidazolderivat der allgemeinen Formel IV durch Abspalten der Aminoschutzgruppe in das Endprodukt der allgemeinen Formel I überführt.

Die Abspaltung der Aminoschutzgruppe erfolgt auf fachmännisch übliche Weise wie beispielsweise durch Zugabe einer organischen Säure wie Trifluoressigsäure oder einer Mineralsäure wie Salzsäure, Schwefelsäure oder Phosphorsäure, gegebenenfalls in Gegenwart einer der zuvor beschriebenen Organometallverbindungen. Je nach Aminoschutzgruppe kann auch eine starke Base wie z.B. Natriumhydroxid verwendet werden. Vorzugsweise wird die Aminoschutzgruppe durch Zugabe einer Mineralsäure abgespalten.

Wie fachmännisch bekannt, ist die Wahl der Reaktionstemperatur und die Wahl des Lösungsmittels von der abzuspaltenden Aminoschutzgruppe abhängig und kann beispielsweise ebenfalls Houben Weyl, ibid, entnommen werden. Wird beispielsweise die zuvor als bevorzugt beschriebene Aminoschutzgruppe Dialkylaminomethyl abgespalten, wird die Abspaltung bei einer Temperatur von 0 bis 100°C, vorzugsweise bei einer Temperatur von 20 bis 50°C, und bei einem pH-Wert von 0 bis 7, vorzugsweise von 2 bis 5, durchgeführt. Zweckmässig erfolgt diese Abspaltung in wässrigem oder organisch/wässrigem Medium. Vorzugsweise erfolgt die Abspaltung durch Zugabe einer organischen Säure oder einer Mineralsäure.

Die Abspaltung von Sulfonylschutzgruppen kann bei Temperaturen von 0 bis 100°C, vorzugsweise von 20 bis 50°C, in wässrigem oder organisch/wässrigem Medium erfolgen. Zweckmässig erfolgt diese Abspaltung unter stark sauren (pH-Wert kleiner 2) oder unter stark basischen (pH-Wert grösser 11) Bedingungen. Dabei wird der pH-Wert zweckmässig entweder durch Zugabe einer organischen Säure, einer Mineratsäure oder durch Zugabe einer starken Base wie Natriumhydroxid eingestellt.
Ein weiterer Bestandteil der Erfindung sind die in der Literatur noch nicht beschriebenen Verbindungen der allgemeinen Formel 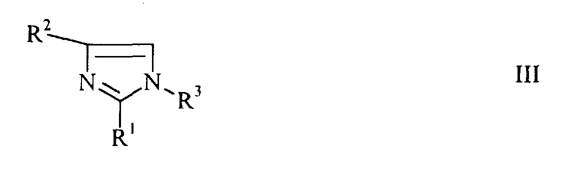 oder deren Tautomere, worin R¹ Alkyl, R² Wasserstoff und R³ eine Aminoschutzgruppe bedeutet, ausgenommen 2-Methyl-3-dimethylaminomethlyimidazol und 2-Methyl-3-piperidinomethylimidazol, sowie die in der Literatur noch nicht beschriebenen Verbidungen der allgemeinen Formel 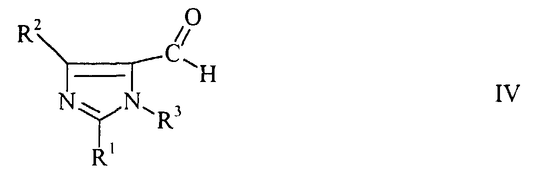 oder deren Tautomere, worin R¹ Alkyl, R² Wasserstoff und R³ eine Aminoschutzgruppe bedeutet.

Bevorzugte neue Verbindungen der allgemeinen Formel III sind 2-Butyl-3-dimethylaminomethylimidazol, 2-Ethyl-3-dimethylaminomethylimidazol, 2-Propyl-3-dimethylaminomethylimidazol, 2-Ethyl-3-piperidinomethylimidazol, 2-Propyl-3-piperidinomethylimidazol, 2-Butyl-3-piperidinomethylimidazol, 1,1-Sulfuryl-di-2-methylimidazol, 1,1-Sulfuryl-di-2-ethylimidazol, 1,1-Sulfuryl-di-2-propylimidazol, 1,1-Sulfuryl-di-2-butylimidazol, 2-Methyl-3-dimethylaminosulfonylimidazol, 2-Ethyl-3-dimethylaminosulfonylimidazol, 2-Propyl-3-dimethylaminosulfonylimidazol und 2-Butyl-3-dimethylaminosufonylimidazol.

Bevorzugte Verbindungen der allgemeinen Formel IV sind 2-Methyl-3-dimethylaminosulfonylimidazol-4-carbaldehyd, 2-Ethyl-3-dimethylaminosulfonylimidazol-4-carbaldehyd, 2-Propyl-3-dimethylaminosulfonylimidazol-4-carbaldehyd, 2-Butyl-3-dimethylaminosulfonylimidazol-4-carbaldehyd, 2-Methyl-3-dimethylaminomethylimidazol-4-carbaldehyd, 2-Ethyl-3-dimethylaminomethylimidazol-4-carbaldehyd, 2-Propyl-3-dimethylaminomethylimidazol-4-carbaldehyd, 2-Butyl-3-dimethylaminomethylimidazol-4-carbaldehyd, 2-Methyl-3-piperidinomethylimidazol-4-carbaldehyd, 2-Ethyl-3-piperidinomethylimidazol-4-carbaldehyd, 2-Propyl-3-piperidinomethylimidazol-4-carbaldehyd und 2-Butyl-3-piperidinomethylimidazol-4-carbaldehyd.

### Beispiele:

### Beispiel 1:

### Herstellung von 2-Butyl-3-dimethylaminomethylimidazol

a) 2,48 g Butylimidazol wurden in 4 ml Wasser vorgelegt, mit 1,66 g Dimethylaminhydrochlorid versetzt und unter leichtem Kühlen mit konz. Salzsäure von pH 9,0 auf pH 4,9 gestellt. Man gab 1,82 g Formalin (36% Formaldehyd in Wasser) zu und liess 72 h bei Raumtemperatur rühren. Es wurde mit 30% NaOH basisch gestellt, mit Essigsäureethylester extrahiert und die organische Phase eingeengt. Man erhielt 2,69 g Produkt als gelbes Oel. Die Ausbeute betrug 74%.
b) 12,4 g Butylimidazol und 20,4 g Bis(dimethylamino)methan wurden in 100 ml Tetrahydrofuran während 24 h zum Rückfluss erhitzt. Das Lösungsmittel wurde verdampft und man erhielt 18,64 g Produkt. Die Ausbeute war quantitativ.

1H-NMR (CDCl₃):
   - 6.94 ppm: (s, 1H)
   - 6.87 ppm: (s, 1H)
   - 4.45 ppm: (s, 2H)
   - 2.70 ppm: (t, 2H)
   - 2.26 ppm: (s, 6H)
   - 1.71-1.80 ppm: (m, 2H)
   - 1.38-1.46 ppm: (m, 2H)
   - 0.94 ppm: (t, 3H)

### Beispiel 2:

### 2-Butyl-3-piperidinomethylimidazol

18,6 g Butylimidazol und 12,8 g Piperidin wurden in 45 ml Wasser vorgelegt und unter leichtem Kühlen mit 30 g konz. Salzsäure versetzt. Man gab 15 g Formalin (36 % Formaldehyd in Wasser) zu und liess 44 h bei Raumtemperatur rühren. Es wurde mit 30% NaOH basisch gestellt, mit Essigsäureethylester extrahiert und die organische Phase eingeengt. Man erhielt 33,6 g Produkt als gelbes Oel. Die Ausbeute war quantitativ.
1H-NMR (CDCl₃):
   - 7.00 ppm: (s, 1H)
   - 6.72 ppm: (s, 1H)
   - 4.52 ppm: (s, 2H)
   - 2.63 ppm: (t, 2H)
   - 2.35-2.40 ppm: (m, 4H)
   - 1.59-1.68 ppm: (m, 2H)
   - 1.42-1.50 ppm: (m, 4H)
   - 1.28-1.38 ppm: (m, 4H)
   - 0.89 ppm: (s, 3H)

### Beispiel 3:

### 2-Butyl-3-dimethylaminosulfonylimidazol

12,4 g Butylimidazol und 13,3 g N,N-Dimethylsulfaminsäurechlorid wurden in 200 ml Methylenchlorid vorgelegt und bei Raumtemperatur mit 9,7 g Triethylamin versetzt. Man rührte das Gemisch 20 h bei Raumtemperatur und 2 h bei 40°C, gab 200 ml Wasser zu und trennte die Phasen. Die organische Phase wurde mit Wasser gewaschen, mit Na₂SO₄ getrocknet und eingeengt. Man erhielt 22,2 g Produkt in Form eines roten Oels. Die Ausbeute war 96%.
1H-NMR (CDCl₃):
   - 7.20 ppm: (s, 1H)
   - 6.94 ppm: (s, 1H)
   - 2.93 ppm: (t, 2H)
   - 2.88 ppm: (s, 6H)
   - 1.78-1.85 ppm: (m, 2H)
   - 1.39-1.48 ppm: (m, 2H)
   - 0.97 ppm: (t, 3H)

### Beispiel 4:

### 2-Butyl-3-dimethylaminosulfonylimidazol-4-carbaldehyd

1,25 g 2-Butyl-3-dimethylaminosulfonylimidazol wurde in 25 ml Tetrahydrofuran gelöst, auf-70°C gekühlt und mit 3,3 ml n-Butyllithium, 1,6 n in Hexan, versetzt. Nach 1 h gab man 0,64 ml Methylformiat zu, erwärmte auf Raumtemperatur und rührte 17 h bei dieser Temperatur Anschliessend wurde aufkonzentriert, mit Methylenchlorid / Wasser versetzt und durch Zugabe von 10%-iger Schwefelsäure neutralisiert. Die organische Phase wurde mit Na₂SO₄ getrocknet und eingeengt. Man erhielt 1,35 g Rohprodukt in Form eines gelben Oels, welches durch Chromatographie über Methylenchlorid / Methanol / Hexan 90:5:5 gereinigt wurde. Es resultierten 0,99 g Produkt. Die Ausbeute betrug 76%.
1H-NMR (CDCl₃):
   - 10.01 ppm: (s, 1H)
   - 7.76 ppm: (s, 1H)
   - 3.71-3.77 ppm: (m, 1H)
   - 3.00-3.02 ppm: (m, 1H)
   - 2.90 ppm: (s, 6H)
   - 1.80-1.88 ppm: (m, 2H)
   - 1.39-1.48 ppm: (m, 2H)
   - 1.92-1.99 ppm: (m, 3H)

### Beispiel 5:

### 2-Butyl-3H-imidazol-4-carbaldehyd

a) 0,53 g 2-Butyl-3-dimethylaminosulfonylimidazol-4-carbaldehyd wurden in 20 ml 1 n Salzsäure 20 h bei Raumtemperatur gerührt. Durch Zugabe von gesättigter NaHCO₃-Lösung wurde neutralisiert, das Produkt mit Essigsäureethylester extrahiert, der Extrakt eingedampft. Man erhielt 0,33 g Rohprodukt. Die Ausbeute war quantitativ.
b) 1 g 2-Butyl-3-dimethylaminomethylimidazol wurde in 25 ml Tetrahydrofuran gelöst, auf-70°C gekühlt und mit 3,9 ml n-Butyllithium 1,6 n in Hexan versetzt. Nach 1 h gab man 0,73 g N,N-Dimethylformamid zu, erwärmte auf Raumtemperatur und rührte 17 h bei dieser Temperatur. Anschliessend wurden 25 ml 1 n Salzsäure zugegeben. Man rührte das Gemisch 15 min, destillierte einen Teil des Lösungsmittels im Vakuum ab, rührte 1 h bei 50°C, neutralisierte mit Natriumbicarbonat und extrahierte mit Methylenchlorid. Der Extrakt wurde eingedampft und man erhielt 0.84 g Rohprodukt in Form eines Oels. Die Ausbeute war quantitativ.

## Patentansprüche

1. Verfahren zur Herstellung von Formylimidazolen der allgemeinen Formel 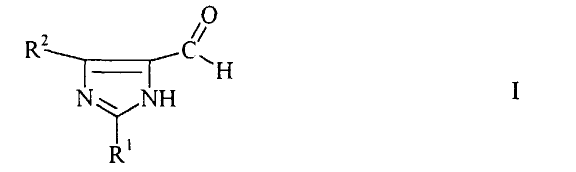 oder deren Tautomeren, worin R¹ Wasserstoff oder Alkyl und R² Wasserstoff, Halogen oder Alkyl bedeutet, dadurch gekennzeichnet, dass man in einer ersten Stufe ein Imidazolderivat der allgemeinen Formel 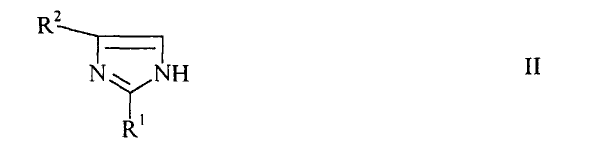 oder dessen Tautomere, worin R¹ und R² die genannte Bedeutung haben, durch Einführen einer Aminoschutzgruppe in ein Imidazolderivat der allgemeinen Formel 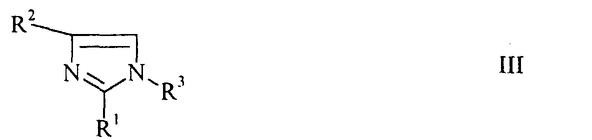 oder dessen Tautomere, worin R³ eine Aminoschutzgruppe bedeutet, überführt, dieses in einer zweiten Stufe in Gegenwart einer Organometall-Verbindung und eines geeigneten Elektrophils in ein Imidazolderivat oder allgemeinen Formel 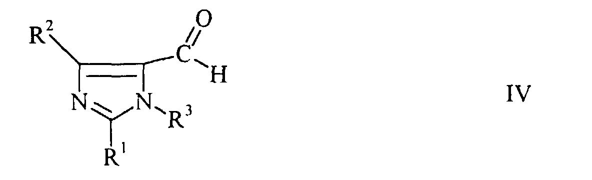 oder dessen Tautomere, worin R¹, R² und R³ die genannte Bedeutung haben, formyliert und dann in einer dritten Stufe durch Abspalten der Aminoschutzgruppe das Endprodukt der Formel I erhält.

2. Verfahren nach Anspruch 1, dadurch gekennzeichnet, dass man die Umsetzung in der ersten Stufe in Wasser oder in einem polar protischen oder polar aprotischen Lösungsmittel durchführt.

3. Verfahren nach einem der Ansprüche 1 oder 2, dadurch gekennzeichnet, dass man als Aminoschutzgruppe Dialkylaminosulfonyl, Dialkylaminomethyl, Piperidinomethyl oder das Reaktionsprodukt von Sulfurylchlorid mit dem entsprechenden Imidazolderivat verwendet.

4. Verfahren nach einem der Ansprüche 1 bis 3, dadurch gekennzeichnet, dass man die Formylierung in der zweiten Stufe in einem aprotischen Lösungsmittel durchführt.

5. Verfahren nach einem der Ansprüche 1 bis 4, dadurch gekennzeichnet, dass man als Organometall-Verbindung in der zweiten Stufe eine Alkylmetall-Verbindung verwendet.

6. Verfahren nach Anspruch 5, dadurch gekennzeichnet, dass man als Alkylmetall-Verbindung eine Alkylalkalimetall-Verbindung verwendet.

7. Verbindungen der allgemeinen Formel 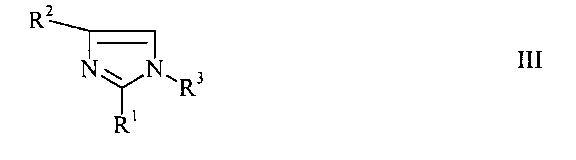 oder deren Tautomere, worin R¹ Alkyl, R² Wasserstoff und R³ eine Aminoschutzgruppe bedeutet, ausgenommen 2-Methyl-3-dimethylaminomethylimidazol und 2-Methyl-3-piperidinomethylimidazol.

8. Verbindungen der allgmeinenen Formel 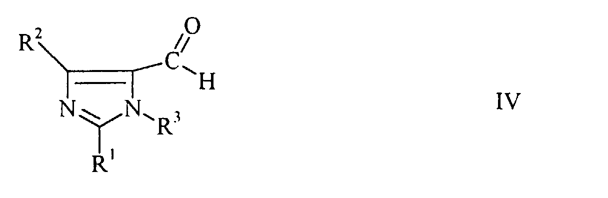 oder deren Tautomere, worin R¹ Alkyl, R² Wasserstoff und R³ eine Aminoschutzgruppe bedeutet.
